# EUROPÄISCHE PATENTSCHRIFT

(11) **EP 2 395 978 B1**
(45) Veröffentlichungstag und Bekanntmachung des Hinweises auf die Patenterteilung: **27.05.2015**
(21) Anmeldenummer: 10710995.1
(22) Anmeldetag: 11.02.2010
(51) Int. Cl.: A61K 9/14, A61K 9/16, A61K 31/00

(54) **VORRICHTUNG UND VERFAHREN ZUR HERSTELLUNG PHARMAZEUTISCH HOCHFEINER PARTIKEL SOWIE ZUR BESCHICHTUNG SOLCHER PARTIKEL IN MIKROREAKTOREN**
DEVICE AND METHOD FOR PRODUCING PHARMACEUTICALLY HIGHLY REFINED PARTICLES AND FOR COATING SAID PARTICLES IN MICROREACTORS
DISPOSITIF ET PROCÉDÉ DE FABRICATION DE PARTICULES PHARMACEUTIQUES ULTRAFINES ET DE REVÊTEMENT DE TELLES PARTICULES DANS DES MICRORÉACTEURS

(30) Priorität: 11.02.2009 DE 102009008478
(43) Veröffentlichungstag der Anmeldung: 21.12.2011
(73) Patentinhaber: Instillo GmbH, 66740 Saarlouis (DE)
(72) Erfinder: TÜRELI, Akif Emre, 66133 Saarbrücken (DE); PENTH, Bernd, 66822 Lebach (DE); LANGGUTH, Peter, 55268 Nieder-Olm (DE); BAUMSTÜMMLER, Bernd, 66740 Saarlouis (DE)
(74) Vertreter: Vièl, Christof
(86) Internationale Anmeldenummer: PCT/DE2010/075015
(87) Internationale Veröffentlichungsnummer: WO 2010/091683

(56) Entgegenhaltungen:
- EP-A1- 1 652 515
- WO-A1-00/38811
- WO-A2-00/61275
- WO-A2-02/055059
- DE-A1- 19 617 085
- US-A1- 2004 173 139

## Beschreibung

Die vorliegende Erfindung betrifft ein Verfahren zur Herstellung von pharmazeutischen Wirkstoffpartikeln mit geringer Partikelgröße.

In den Schutzrechtsanmeldungen US 2003/0206959, US 5,314,506, US 6,558,435, US 7,041,144, DE 102 14 031, DE 10 2005 017 777, DE 10 2005 053 862, DE 10 2005 011 786, DE 196 17 085 sind eine Reihe von in Wasser schwer löslichen, pharmazeutischen Wirkstoffen und Verfahren beschrieben, nach denen die Wirkstoffe in eine nanoskalige Form gebracht werden können um so deren Bioverfügbarkeit zu erhöhen.

Aus der WO 00/38811 A1 sind eine Vorrichtung und ein Verfahren zum Herstellen von kristallinen Partikeln bekannt, bei dem eine durch Ultraschall induzierte Fällung erfolgt.

Die WO 02/055059 A2 beschreibt ein Verfahren zur Herstellung von pharmazeutisch aktiven Verbindungen im Submikrometer-Bereich, wobei eine erste Menge der pharmazeutisch aktiven Substanz in einem wasserlöslichen ersten organischen Lösungsmittel gelöst wird, die hierbei entstehende Lösung mit einem zweiten Lösungsmittel gemischt wird, um die pharmazeutisch aktive Substanz zu fällen und die erste Lösung oder das zweite Lösungsmittel oder die Mischung der Lösung mit dem zweiten Lösungsmittel geimpft wird.

In der US 2004/0173139 A1 werden eine Vorrichtung und ein Verfahren zum Kristallisieren einer Verbindung mittels hydrodynamischer Kavitation beschrieben.

Aus der EP 1 652 515 A1 ist ein Verfahren zur Herstellung von Ultramikropartikeln bekannt, wobei eine Substanz zunächst in einem ersten Lösungsmittel, in dem sie gut löslich ist, gelöst wird, dann die Lösung mit einem zweiten Lösungsmittel, in dem die Substanz schlecht löslich ist, gemischt wird und dann die entstehende Mischung einer Emulgierung unter einem festgelegten Druck unterzogen wird.

Zielsetzung der vorliegenden Erfindung war die Möglichkeit, noch kleinere Partikel herzustellen.

Das vorliegende Verfahren bezieht sich auf die Herstellung der dort beschriebenen und namentlich erwähnten Wirkstoffe, beschränkt sich aber nicht auf diese, sondern ist anwendbar auch auf alle anderen in Wasser schwer löslichen pharmazeutischen Wirkstoffe.

Erfindungsgemäß geschieht dies durch eine Kombination von einer Solvent-Nonsolvent-Fällung des Wirkstoffes in Kombination mit einer in-situ - Sprühverdampfung des Solvents in Anwesenheit eines Oberflächenmodifikators.

Der pharmazeutische Wirkstoff wird dazu zunächst in einem wassermischbaren organischen Lösungsmittel wie Ethanol dispergiert und in der Dispersion zusätzlich ein Oberflächenmodifikator gelöst.

Die Dispersion wird mittels einer Pumpe über eine Rohrleitung unter erhöhtem Druck von bis über ein bar, besser bis über 10 bar, noch besser bis über 50 bar durch eine Düse in den Fällungsreaktor gespritzt. Die Düse des Fällungsreaktors dient dabei gleichzeitig als Druckhalteventil. Die zuführende Rohrleitung ist von außen beheizbar, entweder durch eine elektrische Widerstandsheizung oder durch ein Heizbad, günstigerweise mittels einer spiralförmigen Zuführleitung.

In den Fällungsreaktor wird zusätzlich mittels einer zweiten Pumpe durch eine Rohrleitung ebenfalls unter erhöhtem Druck analog das Nonsolvent, bevorzugt Wasser, über eine zweite Düse eingespritzt. Auch diese zuführende Rohrleitung ist von außen beheizbar, entweder durch eine elektrische Widerstandsheizung oder durch ein Heizbad, günstigerweise mittels einer spiralförmigen Zuführleitung.

Beim Fällungsreaktor handelt es sich um einen Mikroreaktor, der bevorzugt aufgebaut ist, wie in EP 1 165 224 beschrieben. Als Gas wird bevorzugt Stickstoff verwendet.

Durch die Kollision des Wirkstoff - Flüssigkeitsstahls mit einem Wasserstrahl kommt es zu einer Nonsolvent-Fällung und der Bildung eines feinen Nebels. Auf diese Weise wird das Solvent aus dem Solvent-Nonsolvent-Gemisch praktisch zeitgleich mit der Fällung verdampft. Dazu ist es nicht erforderlich, dass die Temperatur des Solvent-Nonsolventgemisches oberhalb der Verdampfungstemperatur des Solvents liegt.

Wegen des hohen Staudruckes der Flüssigkeiten vor den Düsen, den sich daraus ergebenden hohen Strahlgeschwindigkeiten der kollidierenden Flüssigkeitsstrahlen und der sich daraus ergebenden Feinheit der Nebeltropfen, aber auch durch die erhöhte Temperatur zumindest einer der Flüssigkeitsstrahlen und der zusätzlichen Durchspülung des Reaktors mit Luft, Warmluft, Heißluft oder besser einem optional vorerhitzten Inertgas, wie Stickstoff kommt es zu einer sehr schnellen Verdampfung des Lösungsmittels und dadurch zur Bildung sehr kleiner gecoateter Wirkstoffpartikel.

Die Lösung lässt man demnach als feinen erhitzten Flüssigkeitsstrahl aus Lösungsmittel mit zumindest einem zweiten erhitzten Flüssigkeitsstrahl aus Wasser in einer Gasatmosphäre kollidieren.

Die Geschwindigkeit der Flüssigkeitsstrahlen beträgt meist mehr als 1 m/sec, bei einem Druck von über 50 bar sogar mehr als 50 m/sec.

Im Kollisionspunkt der zumindest zwei Strahlen kommt es zu einer sehr schnellen Solvent-Nonsolvent Fällung. Durch die schnelle Mischung kommt es zu einer hohen übersättigung und einer hohen Anzahl von Keimen, die anschließend nur noch wenig wachsen können, da nur noch wenige gelöste Mengenanteile an Wirkstoff dafür zur Verfügung stehen.

Eine Ostwald-Reifung, bei der kleinere Partikelanteile zugunsten größerer Partikel verschwinden, findet nach der beschriebenen Methode nicht statt, da die dazu erforderliche Restlöslichkeit der gefällten Partikel in dem Solvent-Nonsolvent-Gemisch durch die in-situ-Verdampfung des Solventanteiles aus dem Solvent-Nonsolvent-Gemisch in einem extrem kurzen Zeitraum nach der Fällung der Partikel bereits eliminiert ist.

In einer erweiterten Ausführung können pharmazeutische Wirkstoffe, die im Solvent nur mäßig löslich sind, als Dispersion hergestellt und vor der Zuführung bis zur Lösung erhitzt werden. Dabei kann die Lösung auch so erfolgen, dass während der Zuführung zum Reaktor durch ein beheiztes Rohr die Lösungsmitteltemperatur unter Druck über den Siedepunkt des Lösungsmittels bei Raumtemperatur steigt. Auf diese Weise kann die Erhitzungszeit thermoempfindlicher Wirkstoffe verkürzt werden.

Vorteilhafterweise befindet sich der Kollisionspunkt in einem bewegten Gasstrom.

Der Kollisionspunkt befindet sich vorteilhafterweise in einem Kanal, der sich in der Richtung des strömenden Gases weitet. Insbesondere bei höheren Drücken zur Bildung der Flüssigkeitsstrahlen kommt es zur Bildung sehr kleiner nebelartiger Aerosoltröpfchen, aus denen aufgrund ihrer großen Oberfläche zumindest die leichter flüchtigen Solvent-Anteile sehr schnell verdampfen und den pharmazeutischen Wirkstoff entweder als Dispersion oder in Abhängigkeit von der Temperatur der Flüssigkeiten und des Gases und deren Durchsatzmengen ähnlich wie bei einer Sprühtrocknung als Pulver generieren.

Der Gasstrom und der Mikroreaktor sind neben den Zuleitungen für Solvent und Nonsolvent vorteilhafterweise ebenfalls beheizt. Die Temperatur wird so gewählt, dass das pharmazeutische Produkt thermisch nicht geschädigt wird.

Die erreichbare minimale Partikelgröße liegt nach dieser Methode deutlich unter den Partikelgrößen, die mit den Methoden nach dem Stand der Technik erreichbar sind.

Gut geeignet als Lösungsmittel sind Ethylalkohol oder Aceton. Es kommen aber auch andere leichtflüchtige wassermischbare Lösungsmittel wie Methanol, Isopropanol oder Tetrahydrofuran in Frage.

Gut geeignet sind beispielsweise Reaktoren, wie in DE 10 2005 048 201 beschrieben. Es kommen aber auch andere "Freistrahlreaktoren" in Frage, bei denen sich freie Flüssigkeitsstrahlen in einem Gasraum treffen.

Möglich, aber weniger günstig ist es in der Regel, wenn die Strahlen sich nicht direkt treffen, sondern in einer gasdurchspülten Kammer durch Prall gegen die Kammerwände und daraus entstehender Verwirbelung vermischt werden.

Als Gas kommen alle gasförmigen Elemente, insbesondere inerte Gase, aber auch getrocknete Luft, Stickstoff oder Kohlendioxid in Frage.

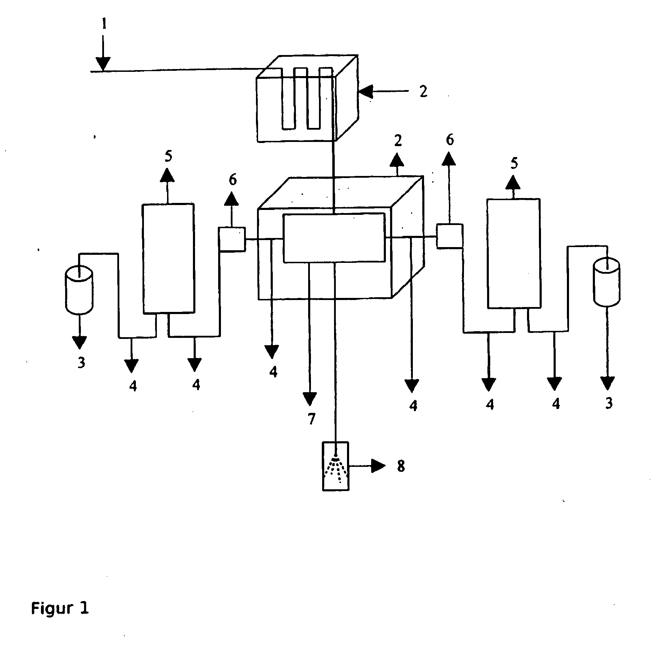

FIG 1 zeigt den Aufbau der erfindungsgemäßen Vorrichtung, 1: Gas zu Leitung, 2: Wasserbad, 3: Vorratsbehälter Solvent, 4: Zuleitungen, 5: Pumpe, 6: Filter, 7: Reaktor, 8: Sammelbehälter, 9: Vorratsbehälter Antisolvent

### Ausführungsbeispiele

### Beispiel 1

Die Wirksubstanz Gliclazid wurde zusammen mit dem Polymer Eudragit S100 in Methanol gelöst, so dass sich eine Gesamtkonzentration von 2 mg/ml und ein Polymer:Wirkstoff Verhältnis von 200 zu 1 ergibt. Zur Herstellung der Nanopartikel wurde ein Stickstoff-Gasstrom mit einem Arbeitsdruck von 0,1 bar eingestellt, die Flussraten des wirkstoff- und polymerhaltigen Methanols wurde auf 0,5, 2,5 und 10 ml/min eingestellt, die Flussrate des als Antisolvent eingesetzten Wassers betrug 10 ml/min.

Hohe Wirkstoffbeladungseffizienten (92,0, 97,6%) konnten erreicht werden indem das Polymer:Wirkstoff Verhältnis auf 200 eingestellt und das Antisolvent / Solvent Flussrate Verhältnis größer als 5 gewählt wurde.

### Beispiel 2

Nanopartikel wurden wie in Beispiel 1 beschrieben hergestellt jedoch unter Verwendung von Danazol als Wirkstoff, Hydroxypropyl Methylcellulosephtalat als Polymer und Aceton als Lösungsmittel. Hohe Wirkstoffbeladungseffizienten bis zu 100% konnten durch die Wahl des Polymer:Wirkstoff Verhältnisses von 20, unabhängig vom Antisolvent / Solvent Flussraten-Verhältnis, erreicht werden.

### Beispiel 3

Nanopartikel wurden wie in Beispiel 1 beschrieben hergestellt jedoch mit einem Polymer: Wirkstoff Verhältnis von 200 bei Temperaturen von 40, 60, 80 und 100 °C unter Verwendung von Stickstoff-Arbeitsdrücken von 0,1 und 1 bar. Es konnte beobachtet werden, dass eine Erhöhung der Temperatur oder des Druckes zu einer Vergrößerung der Nanopartikel führte. Variationen dieser Parameter ermöglichten die Herstellung von Nanopartikel im Größenbereich von 205 bis 756 nm.

### Beispiel 4

Nanopartikel wurden wie im Beispiel 2 beschrieben hergestellt, wobei ein Polymer:Wirkstoff Verhältnis von 20 bei unterschiedlichen Temperaturen von 40, 60, 80 und 100 °C und Stickstoff-Arbeitsdrücke von 0,1 und 1 bar verwendet wurden. Es konnte beobachtet werden, dass eine Erhöhung der Temperatur oder des Druckes zu einer Vergrößerung der Nanopartikel führte. Variationen dieser Parameter ermöglichten die Herstellung von Nanopartikel im Größenbereich von 30 bis 275 nm.

### Beispiel 5

Nanopartikel wurden wie im Beispiel 1 beschrieben hergestellt wobei ein Polymer:Wirkstoff Verhältnis von 200 bei unterschiedlichen Feststoff-Gesamtkonzentrationen von 2, 3, 5 und 8 mg/ml in Methanol, Aceton oder Tetrahydrofuran verwendet wurden. Es konnte beobachtet werden, dass eine Erhöhung des Feststoff-Gesamtgehaltes zu einer Vergrößerung der Nanopartikel führte. Es ergab sich eine Vergrößerung des mittleren Durchmessers mit Verwendung der verschiedenen Lösungsmittel in der Reihenfolge MeOH>THF>Aceton. Variationen dieser Parameter ermöglichten die Herstellung von Nanopartikel im Größenbereich von 70 bis 300 nm.

### Beispiel 6

Nanopartikel wurden wie im Beispiel 2 beschrieben hergestellt wobei ein Polymer:Wirkstoff Verhältnis von 50 bei unterschiedlichen Feststoff-Gesamtkonzentrationen von 3, 5 und 8 mg/ml in Aceton:Ethanol 50:50 (w/w) oder Ethanol:Wasser 90:5 (w/w) Mischungen verwendet wurden. Es konnte beobachtet werden, dass eine Erhöhung des Feststoff-Gesamtgehaltes zu einer Vergrößerung der Nanopartikel führte. Es ergab sich eine Vergrößerung des mittleren Durchmessers mit Verwendung der verschiedenen Lösungsmittel-Gemische in der Reihenfolge EtOH:Wasser > Aceton:EtOH. Variationen dieser Parameter ermöglichten die Herstellung von Nanopartikel im Größenbereich von 38 bis 325 nm.

## Patentansprüche

1. Verfahren zur Herstellung von pharmazeutischen Wirkstoffpartikeln mit geringer Partikelgröße, **gekennzeichnet durch** folgende Verfahrensschritte:
- Dispergieren von pharmazeutischen Wirkstoffpartikel in einem wassermischbaren Lösungsmittel
- Förderung der so hergestellten Dispersion mittels einer Pumpe unter erhöhtem Druck **durch** eine Dispersions-Förderleitung an deren Ende sich eine Düse mit Druckhaltefunktion befindet
- Lösen der dispergierten Wirkstoffpartikel **durch** Erhitzen der Dispersions-Förderleitung auf eine Temperatur oberhalb der Siedetemperatur des Lösungsmittels bei Normaldruck
- Durchtritt der Wirkstofflösung **durch** die Düse eines Fällungs-Sprühreaktors
- Kollision des Flüssigkeitsstrahles der Wirkstofflösung mit einem **durch** eine weitere Düse des Fällungs-Sprühreaktors gebildeten Flüssigkeitsstrahles, wobei letzterer aus Wasser besteht oder eine wässrige Lösung darstellt, die optional bioverträgliche Inhaltsstoffe enthält, welche eine Oberflächenmodifikation der bei der Fällung entstehenden Feinstpartikel bewirken
- Aufrechterhaltung eines Gasraumes im Kollisionspunkt der Flüssigkeitsstrahlen durch Freiblasen des Fällungsraumes mittels Gaszufuhr oder zumindest teilweises Verdampfen von Lösungsmittel und Wasser im Kollisionsraum **durch** den Druckabfall nach dem Durchtritt **durch** die jeweiligen Düsen oder **durch** Entfernung des entstehenden Dispersionsnebels mittels Schwerkraft bei Verwendung eines Freistrahlreaktors, also eines Reaktors, bei dem der Kollisionsraum hinreichend groß ist
- Extrem schnelle Mischung mit Mischzeit unter 100 msec, bevorzugt unter 1 msec durch das Mischen als "Impinging jets" in einer Gasatmosphäre
- Entstehen nanopartikulärer Keime **durch** eine sehr schnelle diffusionskontrollierte Solvent-Nonsolvent-Fällung im Kollisionspunkt und der tellerartigen Mischzone der Flüssigkeitsstrahlen in einer Gasatmosphäre.

2. Verfahren nach dem voranstehenden Anspruch, **gekennzeichnet dadurch, dass** die Dispersions-Förderleitung einen zusätzlichen von einer Pumpe gespeisten Eingang für reines Lösungsmittel besitzt, wobei das Lösungsmittel ebenfalls eine Temperatur oberhalb der Siedetemperatur des Lösungsmittels bei Normaldruck besitzt und diese Temperatur oberhalb der Temperatur der Dispersions-Förderleitung liegt und dadurch zu einer schnellen Lösung der restlichen noch ungelösten dispergierten Partikel in der geförderten Dispersion führt und dadurch der Wirkstoff thermisch über einen kürzeren Zeitraum belastet wird.

## Claims

1. Method for producing pharmaceutical drug particles of small particle size, **characterised by** the following steps:
- Dispersing pharmaceutical drug particles in a water-miscible solvent
- Pumping the thus-prepared dispersion under raised pressure through a dispersion conveyor line at the end of which a nozzle that functions as a pressure regulator is located
- Dissolving the dispersed drug particles by heating the dispersion conveyor line to a temperature above the boiling point of the solvent at normal pressure
- Passage of the drug solution through the nozzle of a precipitation/spray-drying reactor
- Collision of the liquid jet of drug solution with a liquid jet formed by another nozzle of the precipitation/spray-drying reactor, the latter jet consisting of water or an aqueous solution optionally containing biocompatible constituents that modify the surface of the ultrafine particles formed during precipitation
- Maintenance of a gaseous atmosphere at the collision point of the liquid jets by supplying gas to blow the precipitation zone free, or by at least partial vaporisation of solvent and water in the collision zone as a result of the pressure drop following the passage of the jets through the respective nozzles, or, where a free-jet reactor is used, i.e. a reactor with a sufficiently large collision zone, by gravity-based removal of the dispersion mist
- Extremely rapid mixing due to mixing taking place in the form of impinging jets in a gaseous atmosphere, with a mixing time of less than 100 ms, preferably less than one ms
- Formation of nanoparticulate nuclei by very fast diffusion-controlled solvent/non-solvent precipitation at the collision point and the plate-like mixing zone of the liquid jets in a gaseous atmosphere.

2. Method according to the preceding claim, **characterised in that** the dispersion conveyor line has an additional, pump-fed input for pure solvent, the temperature of the solvent being higher than the solvent's boiling point at normal pressure and higher than the temperature of the dispersion conveyor line, thus causing rapid solution of the remaining still-undissolved dispersed particles in the dispersion being conveyed and reducing the time during which the drug is under thermal stress.

## Revendications

1. Procédé pour la fabrication de particules de substances actives pharmaceutiques ayant une faible taille de particule, **caractérisé par** les étapes suivantes :
- dispersion de particules de substances actives pharmaceutiques dans un solvant miscible à l'eau
- refoulement de la dispersion ainsi obtenue au moyen d'une pompe sous pression élevée à travers une conduite de transport de dispersion à l'extrémité de laquelle se trouve une buse ayant une fonction de maintien de la pression
- dissolution des particules de substances actives dispersées par chauffage de la conduite de transport de dispersion à une température supérieure à la température d'ébullition du solvant a pression normal
- passage de la solution de substances actives à travers la buse d'un réacteur à pulvérisation et à précipitation
- collision du jet de liquide de la solution de substances actives avec un jet de liquide formé par une autre buse du réacteur à pulvérisation et à précipitation, ce dernier jet se composant d'eau ou constituant une solution aqueuse qui contient de façon optionnelle des composants biocompatibles qui entraînent une modification de surface des particules extrêmement fines obtenues par la précipitation
- maintien d'un espace gazeux au point de collision des jets de liquides en purgeant l'espace de précipitation au moyen d'un apport de gaz ou par vaporisation au moins partielle du solvant et de l'eau dans l'espace de collision en raison de la baisse de pression après le passage à travers les buses respectives ou par élimination du brouillard de dispersion formé au moyen de la gravité en utilisant un réacteur à libres faisceaux, c'est-à-dire un réacteur dans lequel l'espace de collision est suffisamment grand
- mélange extrêmement rapide avec une durée de mélange inférieure à 100 msec, de préférence inférieure à 1 msec, par mélange à jets contrariés (impinging jets) sous atmosphère gazeuse
- formation de germes de nanoparticules par une précipitation solvant/non-solvant très rapide contrôlée par diffusion au point de collision et dans la zone de mélange en forme d'assiette des jets de liquide sous atmosphère gazeuse.

2. Procédé selon la revendication précédente, **caractérisé en ce que** la conduite de transport de dispersion possède une entrée supplémentaire alimentée par une pompe pour du solvant pur, le solvant possédant lui aussi une température supérieure à la température d'ébullition du solvant à pression normale, et cette température étant supérieure à la température de la conduite de transport de dispersion ce qui conduit à une rapide mise en solution du reste des particules dispersées encore non dissoutes dans la conduite de transport de dispersion de sorte que la substance active est sollicitée thermiquement durant un laps de temps plus court.
